# EUROPEAN PATENT APPLICATION

(11) **EP 4 546 355 A1**
(43) Date of publication of application: **30.04.2025**
(21) Application number: 24207050.6
(22) Date of filing: 16.10.2024
(51) Int. Cl.: G16H 10/60, G06F 21/60, G16H 30/20, G16H 40/67, G06F 21/00, G06F 21/62, H04L 9/40

(54) **MEDICAL INFORMATION PROCESSING METHOD, CLOUD PLATFORM SERVICE SYSTEM OF MEDICAL INFORMATION, AND LOCAL SERVER**

(30) Priority: 24.10.2023 TW 112140676
(71) Applicant: Wang, Fu-Ming, Datong, Taipei City 103 (TW); Wu, Yu-Te, Beitou, Taipei City 112 (TW); Guo, Wan-Yuo, Beitou,Taipei City 112 (TW)
(72) Inventor: WANG, Fu-Ming, 103 Datong Dist., Taipei City (TW)
(74) Representative: Zimmermann, Tankred Klaus

(57) **Abstract**

A medical information processing method, which is used to process medical information including descriptive information and to-be-processed data. The processed medical information conforms a calculation of a medical information calculation application. The method includes a calculation required information detection process and a verification information generation process. The calculation required information detection process detects at least one calculation required information required by the medical information calculation application from the descriptive information, if the detection result is true, deletes all or part of accompanying descriptive information other than the calculation required information, and generates to-be-processed medical information based on the to-be-process data and the remained descriptive information. The verification information generation process generates a verification information when the to-be-processed medical information is generated by the calculation required information detection process.

## Description

### BACKGROUND

### Technology Field

The present disclosure relates to an information processing method and a cloud platform service system, and in particular, to a medical information processing method and a cloud platform service system of medical information.

### Description of Related Art

With the development and popularization of cloud technology, cloud platform services have gradually developed into three major service models in recent years. One of these service models is Software as a Service (SaaS). SaaS is a cloud platform service that provides applications to users. A simple example of SaaA is Gmail. Through the Gmail cloud platform service, users can use email services without any preparation other than connecting to Internet. Furthermore, with the development of cloud platform services, by operating data or applications on the cloud (Internet), people's use of all software services is no longer limited by time and location.

In addition, with the vigorous developments of AI model calculation technology and analysis technology for medical image information, physiological signal information, or diagnosis-related text information (hereinafter collectively referred to as medical information), as well as the actual demand trends of various medical units and users, how to develop medical information AI model calculation applications or analysis applications into SaaS platforms has become a popular topic in the medical information processing and analysis service industry.

However, in SaaS cloud platform services, the issues that are most valued and concerned by patients, medical units, or government units include, for example, information security or personal privacy, and these issues are even more important for medical-related information. Therefore, almost all countries have special legislation to strictly regulate it by for example, personal information laws or health care laws. In other words, if services such as medical information AI model calculations or medical information analysis are developed into SaaS cloud systems, there will of course be concerns about personal information leakage or other medical information security issues.

As mentioned above, regarding the cloud platform service system for AI model calculations or analysis of medical information, the main services are focused on the transmissions of medical information, analysis of medical information or AI model calculations of medical images. Therefore, in order to solve the above-mentioned information security or personal privacy issues, the first issue to be solved is the information security and privacy in the transmission of medical information.

However, in addition to the commonly used DICOM file, the file formats of currently common medical information include RTSS files, NIfTI files, Analyze files, and the likes. These file formats are different from each other, and the contained information contents thereof are also different. Therefore, it is desired to provide a medical information processing method and a cloud platform service system that have a de-identification verification function for various medical information file formats, so that the patient's medical information can comply with relevant information security requirements and ensure patient privacy during the transmission or calculation process of the cloud platform service system.

Moreover, different AI model calculation applications or analysis applications are needed for responding to the medical information of various diseases of patients. In other words, in the future medical information cloud platform service system, it can be expected that numerous AI model calculation applications or analysis applications will be provided. However, since the necessary information for calculations required by various AI model calculation applications or analysis applications are different. Therefore, when the medical information is uploaded to the cloud for calculation, if the uploaded medical information lacks calculation-related information, the cloud applications cannot calculate normally or even obtain incorrect calculating results. Thus, it is desired to provide a medical information processing method and a cloud platform service system of medical information that have the medical information checkout function for these medical information file formats to ensure that cloud calculations can proceed smoothly and produce correct results.

Therefore, it is desired to provide a medical information processing method and a cloud platform service system of medical information that have the checkout function for the calculation required medical information and the security function for personal information and personal privacy.

### SUMMARY

An objective of this disclosure is to provide a medical information processing method and a cloud platform service system of medical information that have the checkout function for the calculation required medical information and the security function for personal information and personal privacy.

To achieve the above, the present disclosure provides a medical information processing method, which is used to process medical information containing descriptive information and to-be-processed data, wherein the medical information is processed to conform a calculation of a medical information calculation application. The medical information processing method includes a calculation required information detection process and a verification information generation process. The calculation required information detection process is configured to detect at least one calculation required information required by the calculation of the medical information calculation application from the descriptive information of the medical information. If a detection result of the calculation required information detection process is true, the calculation required information detection process is further configured to delete all or part of accompanying descriptive information, which is a residual part of the descriptive information other than the calculation required information, and to generate to-be-processed medical information based on the to-be-processed data and the remained descriptive information. The verification information generation process is configured to generate a verification information when the to-be-processed medical information is generated in the calculation required information detection process.

In one embodiment, the accompanying descriptive information comprises direct personal information and indirect personal information.

In one embodiment, the medical information calculation application comprises a medical information AI model calculation application or a medical information analysis application.

In one embodiment, the descriptive information and the to-be-processed data are stored in one single data file or in separated data files.

In one embodiment, the to-be-processed medical information is a DICOM file, a RTSS file, a NIfTI file, an ANALZE file, a JPG file, a Tiff file, or a Bmp file.

In one embodiment, the to-be-processed medical information is an EDF file, a Brain Vision Analyzer file, a BDF file, a common sports biomechanical data file, or an ASCII text file.

In one embodiment, the to-be-processed data is medical image data, physiological signal data, or non-file descriptive text data.

To achieve the above, the present disclosure further provides a cloud platform service system of medical information, which is applied with an Internet and is used to process the medical information containing descriptive information and to-be-processed data, wherein the medical information is processed to conform a calculation of a medical information calculation application. The cloud platform service system includes a local server and a cloud platform. The local server includes a calculation required information detection module and a verification information generation module. The calculation required information detection module detects at least one calculation required information required by the calculation of the medical information calculation application from the descriptive information of the medical information. If a detection result of the calculation required information detection module is true, the calculation required information detection module further deletes all or part of accompanying descriptive information, which is a residual part of the descriptive information other than the calculation required information, and generates to-be-processed medical information based on the to-be-processed data and the remained descriptive information. The verification information generation module generates a verification information when the calculation required information detection module generates the to-be-processed medical information. The cloud platform includes an application server and a network server. The application server at least stores the medical information calculation application. The network server is used to receive the to-be-processed medical information and the verification information from the local server, and after verifying the verification information, to send the to-be-processed medical information to the application server. The medical information calculation application performs an analysis with the to-be-processed medical information and generates analyzed medical information.

In one embodiment, the analyzed medical information and the verification information are transmitted from the network server to the local server through the Internet, and the local server recovers the to-be-processed medical information to the medical information based on the verification information.

In one embodiment, the accompanying descriptive information comprises direct personal information and indirect personal information.

In one embodiment, the medical information calculation application comprises a medical information AI model calculation application or a medical information analysis application.

In one embodiment, the descriptive information and the to-be-processed data are stored in one single data file or in separated data files.

In one embodiment, the to-be-processed medical information is a DICOM file, a RTSS file, a NIfTI file, an ANALZE file, a JPG file, a Tiff file, or a Bmp file.

In one embodiment, the to-be-processed medical information is an EDF file, a Brain Vision Analyzer file, a BDF file, a common sports biomechanical data file, or an ASCII text file.

In one embodiment, the to-be-processed data is medical image data, physiological signal data, or non-file descriptive text data.

To achieve the above, the present disclosure also provides a local server, which is applied with a medical information calculation application for processing medical information containing descriptive information and to-be-processed data. The local server includes a calculation required information detection module and a verification information generation module. The calculation required information detection module detects at least one calculation required information required by a calculation of the medical information calculation application from the descriptive information of the medical information. If a detection result of the calculation required information detection module is true, the calculation required information detection module further deletes all or part of accompanying descriptive information, which is a residual part of the descriptive information other than the calculation required information, and generates to-be-processed medical information based on the to-be-processed data and the remained descriptive information. The verification information generation module generates a verification information when the calculation required information detection module generates the to-be-processed medical information.

In one embodiment, the accompanying descriptive information comprises direct personal information and indirect personal information.

In one embodiment, the medical information calculation application comprises a medical information AI model calculation application or a medical information analysis application.

In one embodiment, the descriptive information and the to-be-processed data are stored in one single data file or in separated data files.

In one embodiment, the to-be-processed medical information is a DICOM file, a RTSS file, a NIfTI file, an ANALZE file, a JPG file, a Tiff file, or a Bmp file.

In one embodiment, the to-be-processed medical information is an EDF file, a Brain Vision Analyzer file, a BDF file, a common sports biomechanical data file, or an ASCII text file.

In one embodiment, the to-be-processed data is medical image data, physiological signal data, or non-file descriptive text data.

### BRIEF DESCRIPTION OF THE DRAWINGS

The disclosure will become more fully understood from the detailed description and accompanying drawings, which are given for illustration only, and thus are not limitative of the present disclosure, and wherein:
FIG. 1 is a schematic diagram showing an architecture of a cloud platform service system of medical information according to an embodiment of this disclosure;
FIG. 2 is a block diagram of a part of the local server according to an embodiment of this disclosure;
FIG. 3 is a schematic diagram showing the profile of medical information according to an embodiment of this disclosure;
FIG. 4 is a schematic diagram showing the content of medical information according to an embodiment of this disclosure;
FIG. 5 is a schematic diagram showing a table including various calculation required information corresponding different medical information calculation applications;
FIG. 6 is a schematic diagram showing the profile of to-be-processed medical information according to an embodiment of this disclosure; and
FIG. 7 is a flow chart of a medical information processing method according to an embodiment of this disclosure.

### DETAILED DESCRIPTION OF THE DISCLOSURE

The present disclosure will be apparent from the following detailed description, which proceeds with reference to the accompanying drawings, wherein the same references relate to the same elements.

Before discussing preferred embodiments of the present disclosure, in order to avoid redundancy and confusion in word interpretation, some terms used in the specific implementations of the present disclosure are defined and explained as follow.

The term "medical information" includes descriptive information and to-be-processed data. Descriptive information includes personal information used to describe patients, information related to medical units, tests, etc., or information used to describe parameters related to medical images or physiological signal, etc. The to-be-processed data indicates medical image data (e.g. pixel data such as MRI, CT, X-ray, or etc.), physiological signal data (e.g. EEG, ECG, EMG, etc.), or non-file descriptive text data (e.g. disease diagnosis data). In addition, the descriptive information and the to-be-processed data are stored in the same file or are stored in separated files respectively, and the file format of the to-be-processed data can be DICOM file, RTSS file, NIfTI file, ANALZE file, JPG file (or JPEG file), Tiff file, or Bmp file. In addition, the file format of the to-be-processed data can be EDF (European Data Format) file, BrainVision Analyzer file, BDF (Biosemi Data Format) file, common sports biomechanical data file, or ASCII text file.

The term "medical information" or "to-be-processed medical information" can refer to a single piece of information or multiple pieces of temporally related information. The term "medical information calculation application" includes an application that uses an AI model to analyze and calculate the medical information or an application that uses other mathematical calculations or feature calculations to analyze and calculate the medical information.

In this invention, the term "local server" refers to any of servers or computerrelated equipment that exist in various types of hospitals, health examination centers, or medical-related enterprises. The term "cloud" or "cloud platform" refers to any of various servers remotely set up with respect to the local server, such as network servers, application servers, database servers, etc. These servers can exist across countries or be dispersed.

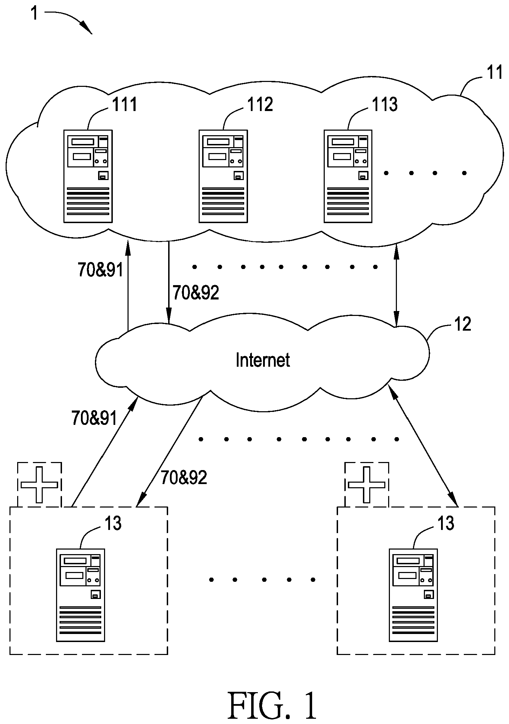

A preferred embodiment of the present disclosure will be specifically described below with reference to drawings. As shown in FIG. 1, the cloud platform service system 1 of medical information of this embodiment mainly includes a cloud platform 11, an Internet 12, and at least one local server 13. The cloud platform 11 can be composed of a plurality of servers configured based on various functional requirements, including a network server 111, an application server 112, a database server 113, and/or any of other servers. In this embodiment, the local server 13 refers to any of the servers or computer equipment installed in various types of hospitals, health examination centers, or medical-related enterprises or units. The dotted lines shown in FIG. 1 represent various types of hospitals, health examination centers, or medical-related enterprises or units. To be noted, the cloud platform 11 can be a public cloud or a private cloud. Specifically, the application server 112 of the cloud platform 11 of this embodiment can be set up remotely with respect to the local server 13, or in the form of a private cloud configured in any of various types of hospitals, health examination centers, or medical-related enterprises or units.

The application server 112 of the cloud platform 11 at least stores at least one medical information calculation application. The medical information calculation application includes the application that uses an AI model to analyze and calculate the medical information, or an application that uses a mathematical calculation or feature calculation other than AI model to analyze and calculate the medical information.

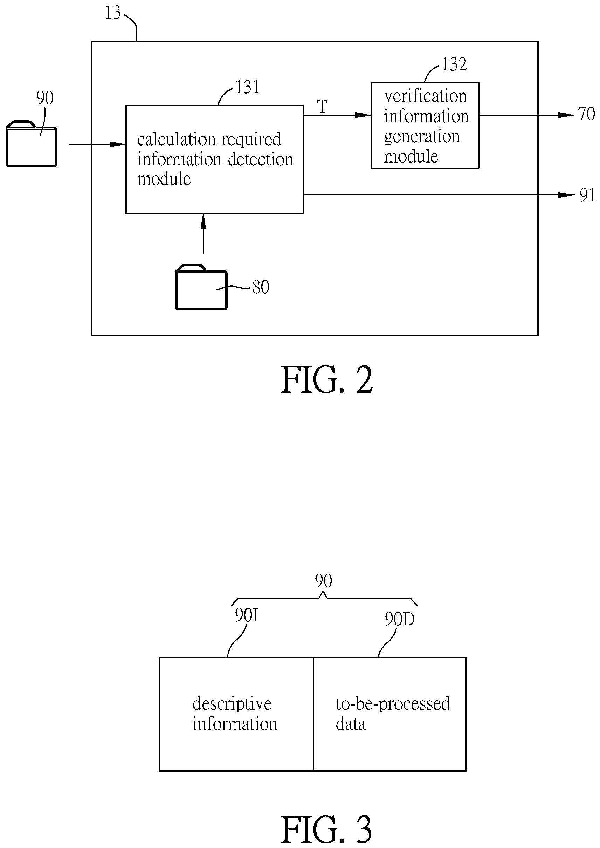

As shown in FIG. 2, the local server 13 includes a calculation required information detection module 131 and a verification information generation module 132. The calculation required information detection module 131 is used to detect and screen out the calculation required information from the medical information 90 that already exists in the local server 13 or the medical information 90 received from a system such as PACS.

The calculation required information detection module 131 uses a calculation required information look-up table 80 of a medical information calculation application pre-stored in the local server 13 to detect calculation required information required by the calculation of the medical information calculation application from the descriptive information of the medical information. In this case, if the calculation required information detection module 131 detects that the items and contents of the calculation required information according to the calculation required information comparison table 80 are correct, it means that the detection result is true (T). When the detection result is true (T), the calculation required information detection module 131 deletes all or part of accompanying descriptive information, which is a residual part of the descriptive information other than the calculation required information, and to generate to-be-processed medical information 91 based on the to-be-processed data and the remained descriptive information (after the deletion). To be noted, in this preferred embodiment, the term "accompanying descriptive information" may include direct personal information and/or indirect personal information of the patient. In other words, as can be seen from this embodiment, when all or part of the accompanying descriptive information other than the calculation required information is deleted and only or almost the calculation required information is remained, the patient's privacy information can be completely secured, thereby complying all information security regulations.

When the detection result of the calculation required information detection module 131 is true (T) and the calculation required information detection module 131 generates the to-be-processed medical information 91, the verification information generation module 132 generates a verification information 70.

The network server 111 of the cloud platform 11 receives the to-be-processed medical information 91 and the verification information 70 from the local server 13 through the Internet 12. After verifying the verification information 70, the network server 111 transmits the to-be-processed medical information 91 to the application server 112. Then, the medical information calculation application stored in the application server 112 is used to analyze and calculate the to-be-processed medical information 91, thereby generating an analyzed medical information 92. In this embodiment, the analyzed medical information 92 may be information such as labels, tracks, or classifications of medical images, analysis results of physiological signal information, or analysis results of various disease diagnosis information.

As shown in FIG. 1, the cloud platform 11 further includes a database server 113. The database server 113 can be used to store the to-be-processed medical information, the verification information, and the analyzed medical information.

To be noted, the network server 111 can then send the analyzed medical information 92 and the verification information 70 back to the local server 13 via the Internet 12. Afterwards, the local server 13 can use the verification information 70 to recover the originally deleted part of the descriptive information, and further process the to-be-processed medical information 91 to obtain the original medical information 90.

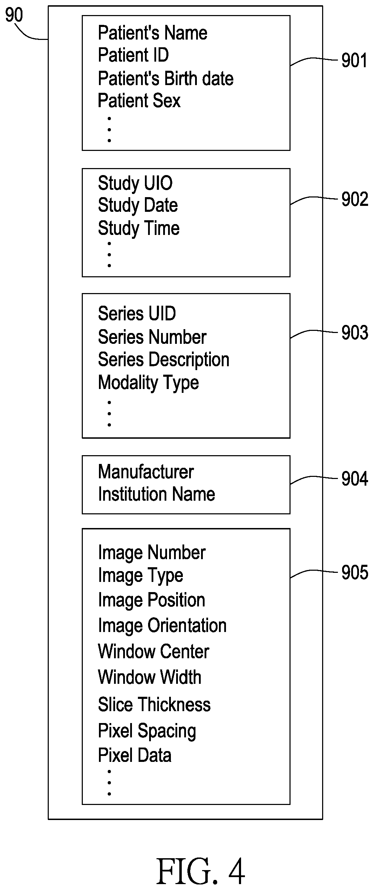

In order to further illustrate the actual operation of the calculation required information detection module 131 and the verification information generation module 132 of the local server 13 of this embodiment, the file format of the medical information 90 is defined in a DICOM file in the following discussion. Generally speaking, as shown in FIG. 3, one medical information 90 in DICOM file mainly includes two parts: descriptive information 90I and to-be-processed data 90D. In this case, the descriptive information 90I is mainly recorded in the header of the DICOM file, and the to-be-processed data 90D refers to pixel data. In addition, as shown in FIG. 4, the content of one medical information file in DICOM file can be roughly divided into five classifications, including patient information 901, study information 902, and series information 903, equipment information 904, and image information 905.

As mentioned above, the patient information may include, for example, patient's name, patient ID, patient's birth date, etc. This information can be obtained from the hospital's HIS or RIS system, or the medical record information filled in during registration, which will be input into the diagnostic instrument by operators later. The study information may include, for example, height, weight, study date, study time, doctor's name, etc. This information is mainly input into the diagnostic instrument by technicians according to the examination report. These contents of this information may change after each interview. The series information may record the parameters used in the equipment during image acquisition, such as series description, modality type, irradiation intensity, etc. The equipment information may include, for example, manufacturer, equipment model, etc. The image information may record the information between the imaging equipment and the obtained image, as well as the information about the image itself, such as window center, window width, image type, image resolution, image depth, and numerical data of the image itself, etc.

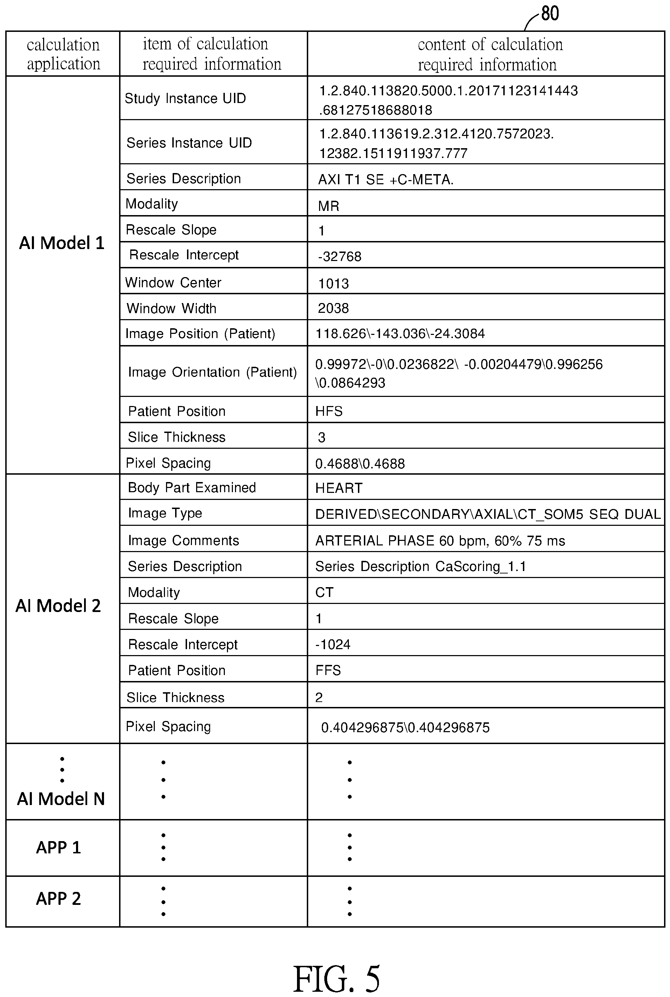

With reference to FIG. 5, the calculation required information detection module 131 can perform the detection process with using a calculation required information comparison table 80 of a medical information calculation application pre-stored in the local server 13. For example, the operator may select one of the calculation applications record in the calculation required information comparison table 80, such as AI model 1 or AI model 2. When selecting the calculation application (e.g. AI model 1 or AI model 2), the required calculation information items and calculation information contents can be found from the calculation required information comparison table 80. Accordingly, the calculation required information detection module 131 can detect the calculation required information (items and contents) required by the calculation of the selected medical information calculation application from the descriptive information 90I of the medical information 90. In this embodiment, if the calculation required information detection module 131 detects that the items and contents of the calculation required information according to the calculation required information comparison table 80 are all correct, it means that the detection result is true (T).

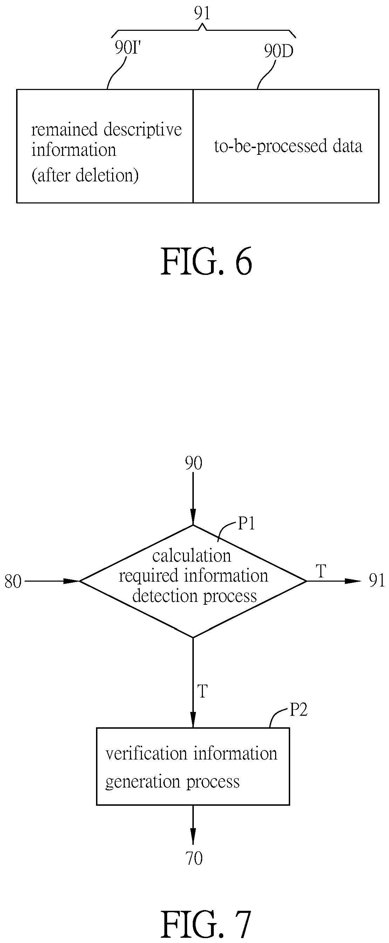

Afterwards, when the detection result is true (T), as shown in FIG. 6, the calculation required information detection module 131 deletes all or part of the accompanying descriptive information, which is the residual part of the descriptive information 90I other than the calculation required information, and generates to-be-processed medical information 91 based on the to-be-processed data 90D and the remained descriptive information 90I'. To be noted, in this embodiment, the information of the remained descriptive information 90I' after the deletion may be equivalent to the calculation information items and the calculation information contents listed in the calculation required information comparison table 80.

Although the above embodiment only discloses the case that the medical information 90 is a DICOM file, this disclosure can be applied to the medical information in any of other file formats, such as NIfTI file, ANALZE file, JPG file (or JPEG file), Tiff file, and Bmp file. In fact, the medical information 90 in any of these other file formats also contains the descriptive information 90I and the to-be-processed data 90D, and the difference between the medical information 90 in the DICOM file format and the medical information 90 in any of these other file formats is that the descriptive information 90I and the to-be-processed data 90D may be stored in one single file or in separated files. Therefore, the cloud platform service system 1 of medical information of this disclosure can be applied to the medical information 90 in any of various file formats.

Moreover, when the detection result of the calculation required information detection module 131 is true (T) and the to-be-processed medical information 91 is generated, the verification information generation module 132 generates the verification information 70. In one embodiment, the verification information 70 can be a set of meaningful verification codes, such as: T00120231010P00001AI001, where "T" represents verified, "001" represents the information transmitting and receiving unit, "P00001" represents a patient No. 00001, and "AI001" represents the selected calculation application. To be noted, in other embodiments, the verification information 70 can be additionally defined according to actual needs.

According to the above description, the cloud platform service system 1 of medical information of this embodiment can generate the to-be-processed medical information 91 by the calculation required information detection module 131 of the local server 13 and generate the verification information 70 by the verification information generation module 132 of the local server 13, wherein the descriptive information of the to-be-processed medical information 91 only remains the calculation-related information. In other words, the to-be-processed medical information 91 uploaded to the cloud no longer contains any direct or indirect personal information about the patient as well as the information related to the inspection unit. Therefore, the cloud platform service system 1 of medical information of this embodiment is a cloud platform service system that has the checkout function for the calculation required medical information and the security function for personal information and personal privacy.

Moreover, although the above embodiment discloses a cloud platform and a local server, the specific descriptions of the cloud platform and the local server of the present invention can be referred to the cloud platform 11 and the local server 13 of the cloud platform service system 1 of the above embodiment. Therefore, the detailed descriptions of the cloud platform and the local server of the present invention will be omitted.

The medical information processing method of the present disclosure will be described hereinafter. For convenience of explanation, the reference numbers used in the following embodiment are the same as those used in the previous embodiment.

As shown in FIG. 7, the medical information processing method of this embodiment includes a calculation required information detection process P1 and a verification information generation process P2.

The calculation required information detection process P1 is configured to detect at least one calculation required information required by the calculation of the medical information calculation application from the descriptive information 90I of the medical information 90. Specifically, in the calculation required information detection process P1, the items and contents of one selected calculation application (e.g. AI model 1), which can be selected from the calculation required information comparison table 80 as shown in FIG. 5, are used to compare with the descriptive information 90I of the medical information 90 so as to detect the calculation required information. If the corresponding items are all existed in the descriptive information 90I and the contents thereof are all correct, the detection result is true (T). Then, the calculation required information detection process P1 is further configured to delete all or part of accompanying descriptive information, which is a residual part of the descriptive information 90I other than the calculation required information, and to generate to-be-processed medical information 91 based on the to-be-processed data and the remained descriptive information 90I'.

The verification information generation process P2 is configured to generate a verification information when the to-be-processed medical information 91 is generated in the calculation required information detection process P1 (i.e., the detection result is true (T)).

As mentioned above, in the medical information processing method of the present disclosure, the calculation required information detection process P1 can compare and detect the descriptive information 90I with the calculation required information comparison table 80, delete all or part of the accompanying descriptive information other than the calculation required information, and generate a to-be-processed medical information 91 based on the remained descriptive information 90I' after deletion and the to-be-processed data 90D. More specifically, the to-be-processed medical information 91 only includes the calculation required information, and the other relevant information about patients or medical units has been removed. Therefore, the medical information processing method of the present invention has the checkout function for the calculation required medical information and the security function for personal information and personal privacy.

Although the disclosure has been described with reference to specific embodiments, this description is not meant to be construed in a limiting sense. Various modifications of the disclosed embodiments, as well as alternative embodiments, will be apparent to persons skilled in the art. It is, therefore, contemplated that the appended claims will cover all modifications that fall within the true scope of the disclosure.

## Claims

1. A medical information processing method, which is used to process medical information containing descriptive information and to-be-processed data, wherein the medical information is processed to conform a calculation of a medical information calculation application, the medical information processing method comprising:
a calculation required information detection process configured to detect at least one calculation required information required by the calculation of the medical information calculation application from the descriptive information of the medical information, wherein if a detection result of the calculation required information detection process is true, the calculation required information detection process is further configured to delete all or part of accompanying descriptive information, which is a residual part of the descriptive information other than the calculation required information, and to generate to-be-processed medical information based on the to-be-processed data and the remained descriptive information; and
a verification information generation process configured to generate a verification information when the to-be-processed medical information is generated in the calculation required information detection process;
wherein, the descriptive information and the to-be-processed data are stored in one single data file or in separated data files.

2. The medical information processing method of claim 1, wherein the accompanying descriptive information comprises direct personal information and indirect personal information.

3. The medical information processing method of claim 1, wherein the medical information calculation application comprises a medical information AI model calculation application or a medical information analysis application.

4. The medical information processing method of claim 1, wherein the to-be-processed medical information is a DICOM file, a RTSS file, an NIfTI file, an ANALZE file, a JPG file, a Tiff file, or a Bmp file.

5. The medical information processing method of claim 1, wherein the to-be-processed medical information is an EDF file, a Brain Vision Analyzer file, a BDF file, a common sports biomechanical data file, or an ASCII text file.

6. The medical information processing method of claim 1, wherein the to-be-processed data is medical image data, physiological signal data, or non-file descriptive text data.

7. A cloud platform service system of medical information, which is applied with an Internet and is used to process the medical information containing descriptive information and to-be-processed data, wherein the medical information is processed to conform a calculation of a medical information calculation application, the cloud platform service system comprising:
a local server comprising a calculation required information detection module and a verification information generation module, wherein the calculation required information detection module detects at least one calculation required information required by the calculation of the medical information calculation application from the descriptive information of the medical information, wherein if a detection result of the calculation required information detection module is true, the calculation required information detection module further deletes all or part of accompanying descriptive information, which is a residual part of the descriptive information other than the calculation required information, and generates to-be-processed medical information based on the to-be-processed data and the remained descriptive information, and the verification information generation module generates a verification information when the calculation required information detection module generates the to-be-processed medical information; and
a cloud platform comprising an application server and a network server, wherein the application server at least stores the medical information calculation application, the network server is used to receive the to-be-processed medical information and the verification information from the local server, and after verifying the verification information, to send the to-be-processed medical information to the application server, and the medical information calculation application performs an analysis with the to-be-processed medical information and generates analyzed medical information;
wherein, the descriptive information and the to-be-processed data are stored in one single data file or in separated data files.

8. The cloud platform service system of claim 7, wherein the analyzed medical information and the verification information are transmitted from the network server to the local server through the Internet.

9. The cloud platform service system of claim 8, wherein the local server recovers the to-be-processed medical information to the medical information based on the verification information.

10. The cloud platform service system of claim 7, wherein the accompanying descriptive information comprises direct personal information and indirect personal information.

11. The cloud platform service system of claim 7, wherein the medical information calculation application comprises a medical information AI model calculation application or a medical information analysis application.

12. The cloud platform service system of claim 7, wherein the to-be-processed medical information is a DICOM file, a RTSS file, an NIfTI file, an ANALZE file, a JPG file, a Tiff file, or a Bmp file.

13. The cloud platform service system of claim 7, wherein the to-be-processed medical information is an EDF file, a Brain Vision Analyzer file, a BDF file, a common sports biomechanical data file, or an ASCII text file.

14. The cloud platform service system of claim 7, wherein the to-be-processed data is medical image data, physiological signal data, or non-file descriptive text data.

15. A local server, which is applied with a medical information calculation application for processing medical information containing descriptive information and to-be-processed data, comprising:
a calculation required information detection module detecting at least one calculation required information required by a calculation of the medical information calculation application from the descriptive information of the medical information, wherein if a detection result of the calculation required information detection module is true, the calculation required information detection module further deletes all or part of accompanying descriptive information, which is a residual part of the descriptive information other than the calculation required information, and generates to-be-processed medical information based on the to-be-processed data and the remained descriptive information; and
a verification information generation module generating a verification information when the calculation required information detection module generates the to-be-processed medical information;
wherein, the descriptive information and the to-be-processed data are stored in one single data file or in separated data files.

16. The local server of claim 15, wherein the accompanying descriptive information comprises direct personal information and indirect personal information.

17. The local server of claim 15, wherein the medical information calculation application comprises a medical information AI model calculation application or a medical information analysis application.

18. The local server of claim 15, wherein the to-be-processed medical information is a DICOM file, a RTSS file, an NIfTI file, an ANALZE file, a JPG file, a Tiff file, or a Bmp file.

19. The local server of claim 15, wherein the to-be-processed medical information is an EDF file, a BrainVision Analyzer file, a BDF file, a common sports biomechanical data file, or an ASCII text file.

20. The local server of claim 15, wherein the to-be-processed data is medical image data, physiological signal data, or non-file descriptive text data.
